# EUROPEAN PATENT SPECIFICATION

(11) **EP 2 921 142 B1**
(45) Date of publication and mention of the grant of the patent: **24.10.2018**
(21) Application number: 14178528.7
(22) Date of filing: 25.07.2014
(51) Int. Cl.: A61F 2/46, A61F 2/44, A61B 17/70, A61B 17/72, A61B 17/74

(54) **Device for bone fixation**
Vorrichtung zur Knochenfixierung
Dispositif de fixation osseuse

(30) Priority: 21.03.2014 TW 103110782
(43) Date of publication of application: 23.09.2015
(73) Proprietor: Spirit Spine Holdings Corporation, Inc., Pasadena, CA 91107 (US)
(72) Inventor: Hsu, Shih-Hsiung, Pasadena, CA 91107 (US); Lin, Kwan Ku, Pasadena, CA 91107 (US); Lin, Shih-Hung, Pasadena, CA 91107 (US); Lu, Shih-Chun, Pasadena, CA 91107 (US)
(74) Representative: de Arpe Fernandez, Manuel

(56) References cited:
- EP-A2- 1 552 797
- WO-A1-2012/054240
- WO-A1-2013/023898
- US-A1- 2006 184 246

## Description

### BACKGROUND OF THE INVENTION

### 1. Field of the Invention

The present invention generally relates to a device for bone fixation. More particularly, the present invention relates to a device for bone fixation that includes an enclosing unit and an expanding unit, and is capable of expanding and contracting.

### 2. The Prior Arts

Following are some common ways of performing a surgery to inject or fill medical fillers into bones:

In a first type of commonly seen surgery, a mechanical-type cavity expansion device (e.g., U.S. Patent Application Publication Nos. 20110196494, 20110184447, 20100076426, 20070067034, 20060009689, 20050143827 & 20020052623, and U.S. Patent Nos. 6,354,995 & 6,676,665) is utilized to expand a cavity in the bone so as to create a space, wherefrom the cavity expansion device is removed after the cavity has been bored. A covering device is then inserted before the process of injecting or stuffing of the medical fillers takes place. This type of surgery has the following drawbacks: the cancellous bone fragments that are crushed by the mechanical-type cavity expansion device often falls into and jamming the mechanical-type cavity expansion device, and causing its components unable to be retrieved, i.e., unable to restore to a contracted state, which in turn causes the entire mechanical-type cavity expansion device to be stuck at the location of the cavity and unable to be removed.

In a second type of commonly seen surgery, a filling-type cavity expansion device (e.g., U.S. patent Nos. 5,972,015, 6,066,154, 6,235,043, 6,423,083, 6,607,544, 6,623,505, 6,663,647, and 6,716,216) is used to bore a cavity in the bone to create a space, where the cavity expansion device is removed from after the cavity is bored. Subsequently, a covering device is placed therein, and the surgery with the injecting or stuffing of the medical filler takes place. In most situations, the filling-type cavity expansion device achieves the effect of cavity expansion first by placing a balloon (the type of balloon used depends on the needs of the surgery) into the bones, then, fills liquid such as water into the balloon with high pressure to expand the balloon, so as to push away the cancellous bone. However, the filling-type cavity expansion device also has some drawbacks. For instance, since the balloon must be attached to a nozzle to operate, when liquid is being filled into the balloon by high pressure, it is possible for the balloon be detached from the nozzle, or, the balloon may even rupture during the filling.

In cases which a cavity expansion process is not done beforehand and a covering device is placed directly into the bone for the insertion of medical fillers, the cavity expansion is instead achieved through the pressure created when medical fillers are inserted into the covering device. The covering device in such cases acts as both a cavity expansion device and a fixation device, as disclosed in Taiwan Patent Application Publication No. 201112995, Taiwan Patent No. 1321467 and U.S. Patent No. 6,248,110. It is also possible to exclude the covering device entirely and instead using a perfusion device to directly pour medical filler at the surgery location to reinforce fixation at the surgery location, as disclosed in U.S. Patent No. 5,514,137. The type of surgery as described above has the following drawbacks: since the cavity is not bored before the surgery, the area for pouring the medical filler cannot be controlled accurately, and the flowing direction of the medical filler could differ from predicted. In addition, there may even be cases that the bones are not properly supported after the pouring of the medical filler is completed, or the medical filler could spread all over the bone, or the medical filler could even seep out from the bone. Furthermore, when using slurry-type medical filler, if the concentration of the medical filler is too dilute, or the particles of the medical filler are too small, it is possible that the medical filler could not properly support the bones, thereby compromising the expected effectiveness of the surgery.

In a third type of commonly seen surgery for bone fixation, a mechanical-type cavity expansion device (e.g., U.S. Patent Application Publication Nos. 20120071977, 20110046739, 20100069913, 20100217335, 20090234398 & 20090005821) is utilized as the bone fixation device and is implanted into and props up the bone with the medical filler being injected therein immediately afterwards to surround the mechanical-type cavity expansion device. When the injecting process is finished, such cavity expansion device is left inside the body along with the medical filler. Due to the lack of a covering device, it is not possible to effectively control the flow direction of the medical filler, and the medical filler can therefore spread out in all directions or even flow out of the bone. Besides, the medical filler also cannot effectively and fully cover the mechanical-type cavity expansion device, and it may leads to that the mechanical-type cavity expansion device gradually contract to a partially expanded state instead of the fully expanded state. Consequently, the bone is not fully propped up, thus losing the original purpose of the bone fixation device.

The applicant of the present invention has filed a device for bone fixation (US patent Application No. US20140114368), in which a covering unit is configured to cover the expanding unit, so as to effectively prevent the crushed cancellous bone from falling into the expanding unit, thus allowing the expanding unit to expand and contract in the bone for the purpose of bone expansion. In addition, when injecting the medical filler, the bone fixation device is able to effectively control the flowing range of the medical filler to prevent the medical filler from spreading all over in the bone. When the injecting process is completed, the medical filler is able to enclose the expanding unit completely, thereby preventing the expanding unit from contracting. However, the structure of the expanding unit of such bone fixation device is rather unitary, since the toughness and collapsing situation of the bones are different for every patient, the expanding unit in such bone fixation device cannot be adjusted to cope with the various situations of different patients. Moreover, it was discovered that the propping effect of such bone fixation device does not meet the expectation when implanted into patients with bones of higher toughness. Therefore, the applicant hereby presents the present invention which improves the abovementioned bone fixation device, so the device for bone fixation can be adapted to use in various situations. A further bone fixation device is disclosed by EP 1 552 797).

### SUMMARY OF THE INVENTION

Based on the above reasons, the present invention provides a device for bone fixation according to claim 1 which utilizes a enclosing unit to enclose the expanding structures of the expanding unit, so as to effectively prevent the crushed cancellous bones from falling into the expanding unit during the cavity expansion process in surgery. In this way, the expanding unit can be expanded and contracted inside the bones to adjust the direction or range of the cavity expansion without being jammed. When medical filler is injected into the bones, the enclosing unit can efficaciously control the range of which the medical filler flows to, so as to prevent the medical filler from spreading all over in the bones. As a result, when the injecting process is completed, the medical filler can fully fill up the expanding unit to keep the expanding structures from contracting. A small amount or a part of the medical filler can exude from the holes of the enclosing unit and bind with the trabecular bones. In addition, the device for bone fixation of the present invention provides multiple expanding structures. With multiple expanding structures, not only the strength of the expanding unit is reinforced for supporting bones with higher toughness, the expanding unit is also able to support bones with different collapsing situations by adjusting the length and arrangement of the expanding structures.

An object of the present invention is to provide a device for bone fixation.

Another objective of the present invention is to provide a device for bone fixation with an enclosing unit.

A further objective of the present invention is to provide a device for bone fixation with an expanding unit.

A further objective of the present invention is to provide a device for bone fixation with an enclosing unit and an expanding unit.

A further objective of the present invention is to provide a device for bone fixation into which the medical filler can be injected via a tube.

A further objective of the present invention is to provide a device for bone fixation which has the capability to prop up a bone.

A further objective of the present invention is to provide a device for bone fixation which can prevent bone fragments from jamming the expanding unit.

A further objective of the present invention is to provide a device for bone fixation in which the medical filler can fully enclose the expanding unit.

A further objective of the present invention is to provide a device for bone fixation in which the expanding unit can be expanded and contracted.

A further objective of the present invention is to provide a device for bone fixation that has multiple expanding structures.

A further objective of the present invention is to provide a device for bone fixation of which the multiple expanding structures have different rib lengths.

A further objective of the present invention is to provide a device for bone fixation in which the ribs of the multiple expanding structures are aligned with each other.

A further objective of the present invention is to provide a device for bone fixation in which the ribs of the multiple expanding structures are staggered from each other.

A further objective of the present invention is to provide a device for bone fixation with a pressing unit.

A further objective of the present invention is to provide a device for bone fixation with a hollow fixing unit.

A further objective of the present invention is to provide a device for bone fixation which has limited control over the extent of medical filler injecting.

For achieving the foregoing objectives, the present invention provides a device for bone fixation that includes: an expanding unit and an enclosing unit. The expanding unit has a first end and a second end, and includes two or more expanding structures capable of switching between an expanded state and a contracted state. The expanding unit includes a first expanding structure located near the first end thereof and a second expanding structure located near the second end thereof. The enclosing unit encloses the expanding unit and has a first end and a second end. The first end of the enclosing unit is secured at the first end of the expanding unit, and the second end of the enclosing unit is secured at the second end of the expanding unit. When the device for bone fixation is placed inside a bone, the expanding structures of the expanding unit are switched to the expanding state and thereby propping up the enclosing unit. Medical filler is filled into the enclosing unit through the second end of the expanding unit.

In the present invention, the expanding unit has a hollowed structure and includes multiple expanding structures. Each expanding structure includes: at least four grooves, disposed circularly on and through the expanding unit about a central axis thereof, thus forming a rib between every two grooves. Each rib has at least one bendable joint.

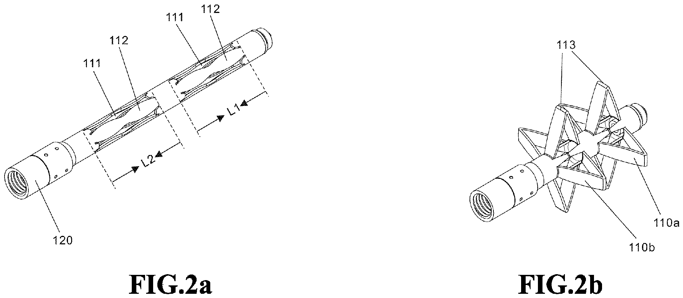

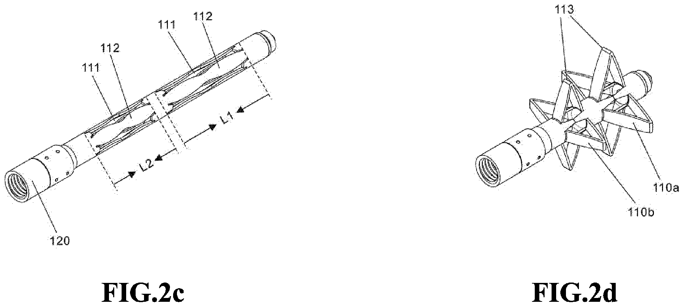

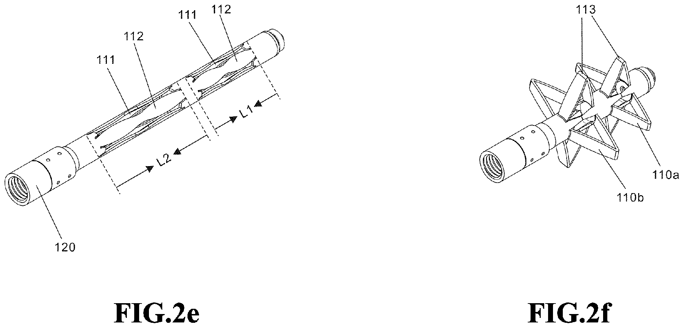

The expanding unit at least includes a first expanding structure and a second expanding structure. The first expanding structure is located on the expanding unit close to the first end thereof, and the second expanding structure is located on the expanding unit close to the second end thereof. Each expanding structure has a rib length. The first expanding structure and the second expanding structure can be configured to have the same rib length (Figs. 2a & 2b), or, one of the first expanding structure and the second expanding structure could have a different rib length than the other (Figs. 2c, 2d, 2e & 2f). In the configuration shown in Figs. 2a∼2f, the ribs of the first expanding structure and the second expanding structures are aligned with each other.

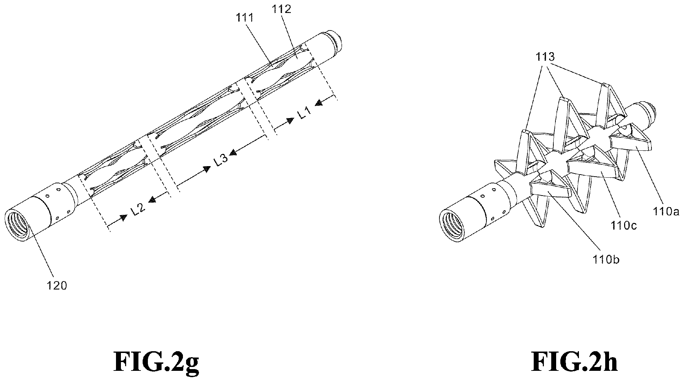

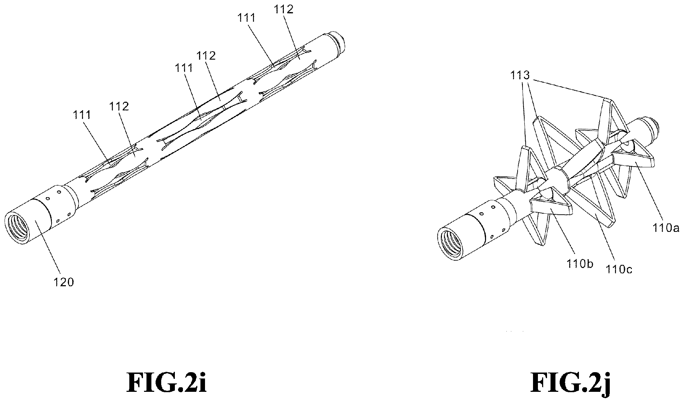

At least one third expanding structure can be disposed between the first expanding structure and the second expanding structure. Similar to the situation described above, the third expanding structure can also be configured to have a rib length different from the rib lengths of the first and second expanding structures, while the ribs of the third expanding structures are aligned with the ribs of the first and second expanding structures (Figs. 2g & 2h). In addition, the ribs of one of the expanding structures can also be configured to be staggered from the ribs of the rest of the expanding structures when viewed from the first end of the expanding unit toward the second end of the expanding unit (Figs. 2i & 2j).

In the above described configurations of the expanding unit, the most preferable configuration is the one having two expanding structures, and the ribs of the two expanding structures are aligned with each other. In addition, the second most preferable configuration is the one having three expanding structures, and the ribs of one of the expanding structures are staggered from the ribs of the rest of the expanding structures.

By providing the expanding structures with the configurations as described above, the device for bone fixation of the present invention is able to reinforce the support strength of the expanding unit, so the expanding structures are less likely to be pushed back to the contracted state from the expanded state when being used to support bones with higher toughness. Furthermore, the supported width of the expanding structures in the expanded state can be adjusted by arranging the rib lengths of each expanding structure, and the expanding direction of the expanding structures can also be adjusted by arranging the location of the ribs. In this way, the user may choose the most fitting expanding unit according to the collapsing shape and status of the bone, and the device for bone fixation provided by the present invention can also support the collapsed bones in a more reliable and steady manner.

In the aforementioned expanding unit, the medical filler is filled into the enclosing unit after the expanding structure props up the enclosing unit in the expanded state, instead of utilizing the pressure generated when the medical filler is injected or stuffed into the enclosing unit to prop up the enclosing unit. Consequently, the situation where the expanding unit and enclosing unit cannot be properly propped up due to insufficient pressure caused by either slurry medical filler being excessively dilute (seeping out via the holes of the enclosing unit), or caused by the particle of the medical filler being too small (falling out from the holes of the enclosing unit) can be avoided. With the configuration provided by the present invention, the function of the expanding unit can be well preserved and reinforced.

The aforementioned enclosing unit can effectively prevent crushed cancellous bone fragments from jamming the expanding structure during the cavity expansion process, thus allowing the expanding structure to be expanded and contracted smoothly; besides, the enclosing unit can effectively control the flowing range of the medical filler to refrain the medical filler from spreading all over within the bones, being injected unevenly or even flowing out of the bone, thereby lowering the risk of the patient being injured during the surgery. When the injecting process is completed, the medical filler can fully fill up and cover the expanding structures, thus lowering the possibility of the expanding structures from contracting.

The aforementioned enclosing unit can have a cylindrical shape, cone-like shape, sphere-like shape, gourd-like shape, cubical shape, cube-like shape or any other conventional shapes when in expanded state.

The aforementioned enclosing unit can be any types of conventional enclosing unit, such as an elastic enclosing unit, mesh-shaped enclosing unit or porous enclosing unit, among which the mesh-shaped enclosing unit and the porous enclosing unit are the preferable choices. The enclosing unit can also be an enclosing unit with a single layer or multiple layers.

The aforementioned enclosing unit is made of biocompatible materials, especially, is made of elastic biocompatible materials, such as poly-urethane (PU), silicone rubber and nylon. Alternatively, the enclosing unit can also be the enclosing units included in similar devices, such as the devices for bone fixation disclosed in U.S. Patent Application Publication Nos. 20040122455, 20040210297, 20040073308 & 20060149379, and U.S. Patent No. 6719773.

Other than elastic and compatible materials, the aforementioned mesh-shaped enclosing unit can also be made of biocompatible surgical sutures, such as catgut and chrome catgut, which are natural and absorbable sutures, silk and cotton threads, which are natural and non-absorbable sutures, polyglycolic acid threads and poly (lactic acid-co-glycolic) ethanol threads, which are synthetic and absorbable sutures, and, nylon threads, polyester fiber threads, polyethylene threads, polypropylene threads, polydioxanone threads and poly-tetra-fluoroethylene threads, which are synthetic and non-absorbable sutures.

The aforementioned mesh-shaped enclosing unit can also be made of biocompatible metal wires, such as titanium, titanium alloy or stainless steel wires.

A protrusion is provided at the first end of the expanding unit for holding against the first end of the enclosing unit. In this way, the enclosing unit can be protected from being damaged by excessively sharp first end of the expanding unit.

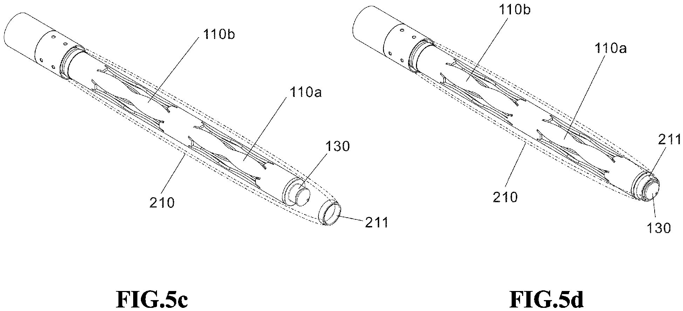

The aforementioned enclosing unit can further include a fixing device for joining with the protrusion of the expanding unit to reinforce the connection between the enclosing unit and the expanding unit. The fixing device can be a ring-shaped fixing member (as shown in Figs. 5c & 5d), or, it can also be any other conventional fixing devices. The fixing device can be connected with the protrusion of the expanding unit by any conventional methods, such as engaging, locking or binding.

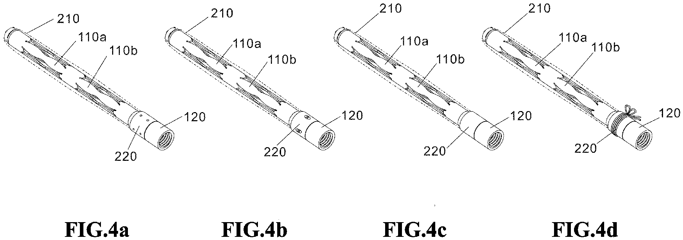

The second end of the aforementioned expanding unit can be connected with the second end of the enclosing unit with any conventional methods, such as riveting, using a retainer ring, locking, adhering, buckling, engaging, or binding. A annular groove can be provided at the second end of the expanding unit, and the enclosing unit can be secured to the annular groove at the second end of the expanding unit by riveting, using a retainer ring, locking or binding (Figs. 4a, 4b, 4c & 4d). In the abovementioned connecting methods, the preferred ones are riveting, using a retainer ring and locking.

The aforementioned medical filler can be any conventional medical filler, such as bone grafts, bone substitutes, bone cement, and/or a mixture, composition and composite of the bone grafts, the bone substitutes and the bone cements. Please see Taiwan Patent Application No. 097141700, Taiwan Patent No. 1227146 and U.S. Patent Application Publication No. 20070088436 for reference. Generally, the aforementioned medical filler can be medical filler that are simply used to fill, such as auto-grafts or allografts. Such medical fillers are implanted bone grafts, and are mixed with bone cements first before they are used in the filling process; however, they have a rather poor solidifying effect. Hence, it is preferable that the medical filler is a medical filler paste that can at least solidify, such as the slurry type medical filler. Alternatively, after the mixture of the bone cement and the transplanted bone grafts are injected in, the slurry that can solidify is injected in to fill up the enclosing unit substantially. Generally speaking, it is best to use only the slurry medical filler. Herein, the slurry type medical filler refers to the slurry which is prepared prior to using or during using, and can solidify within a proper time period after being injected into the enclosing unit. Such medical filler can be bone substitutes, bone cement, and/or a mixture, composition or composite of the two. It is preferable that the aforementioned medical filler is osteo-conductive and/or osteo-inductive. Osteo-conductive medical filler can be conventional HA bone filler, while osteo-inductive medical filler can be conventional SrHA medical filler, among which the osteo-inductive medical filler is the preferred choice. Please see Taiwan Patent Application No. 09714700 for reference. The medical filler mentioned above is well known for a person skilled in the art. In addition to the conventional medical fillers, a combination of conventional medical fillers or modified medical filler may also be used. The medical filler used in the present invention may even be any newly developed medical filler that has the same effect as described.

According to another embodiment, the present invention provides a device for bone fixation, which includes: an expanding unit, an enclosing unit, a hollow joining tube, an auxiliary expanding unit, an operating lever and an injection tool. The expanding unit has a first end and a second end, and includes two or more expanding structures capable of switching between an expanded state and a contracted state. The expanding unit includes a first expanding structure located near the first end thereof and a second expanding structure located near the second end thereof. The enclosing unit encloses the expanding unit and has a first end and a second end. The first end of the enclosing unit is secured at the first end of the expanding unit, and the second end of the enclosing unit is secured at the second end of the expanding unit. When the expanding structures of the expanding unit are switched to the expanding state, the expanding unit props up the enclosing unit. The hollow joining tube has a front end and a rear end. The front end of the hollow joining tube is detachably joined with the second end of the expanding unit. The auxiliary expanding unit has a coupling end and an operating end opposite to the coupling end. The coupling end of the auxiliary expanding unit is detachably coupled to the rear end of the hollow joining tube. The operating lever has a front end and a rear end. The front end of the operating lever is detachably joined with the expanding unit. The rear end of the operating lever is joined with the operating end of the auxiliary expanding unit, thereby allowing the auxiliary expanding unit to adjust the expanding unit to the expanded state or the contracted state by operating the operating lever. The injection tool is joined with the rear end of the hollow joining tube or the rear end of the operating lever, so as to inject or push a medical filler into the enclosing unit via the hollow joining tube or via the operating lever. The auxiliary expanding unit is detached and the injection tool is attached after the auxiliary expanding unit expands the expanding unit with the operating lever for filling the medical filler.

Herein, the expanding unit, enclosing unit and medical filler are the same as those previously described, thus the detailed description thereof will be omitted hereafter.

The aforementioned hollow joining tube can be any conventional hollow joining tube that can achieve the same effect.

The aforementioned auxiliary expanding unit adjusts the expanding structures of the expanding unit to the expanded state or the contracted state with the connection between the auxiliary expanding unit and the operating lever along with the connection between the operating lever and the expanding unit. When the expanding structures are expanded, the auxiliary expanding unit can be detached with the detachable connection between the auxiliary expanding unit and the hollow joining tube. Subsequently, the injection tool is attached for injecting or pouring the medical filler.

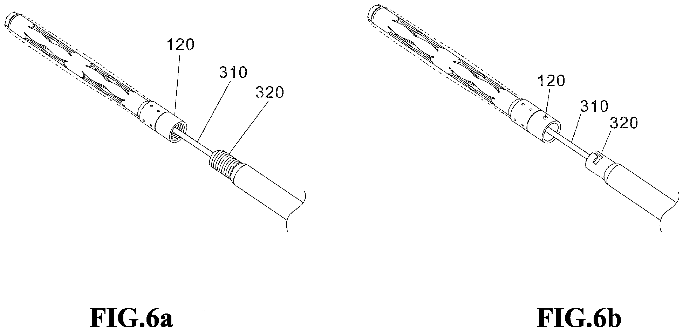

The second end of the aforementioned is detachably attached to the auxiliary expanding unit with any conventional detachably attachment (similar to Figs. 6a & 6b), such as engaging, locking or thread engaging.

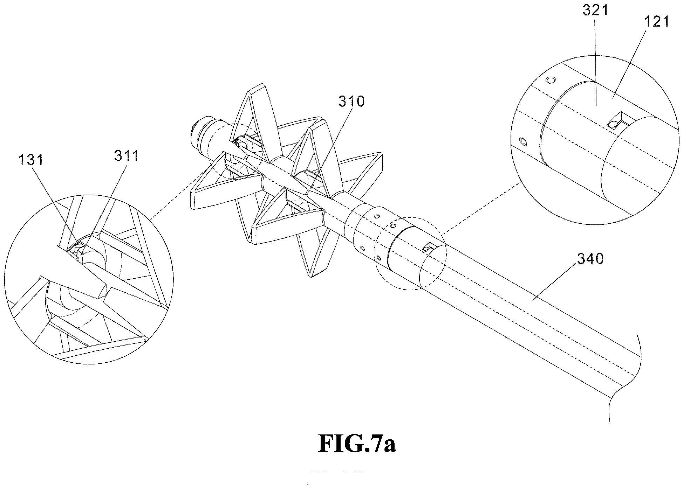

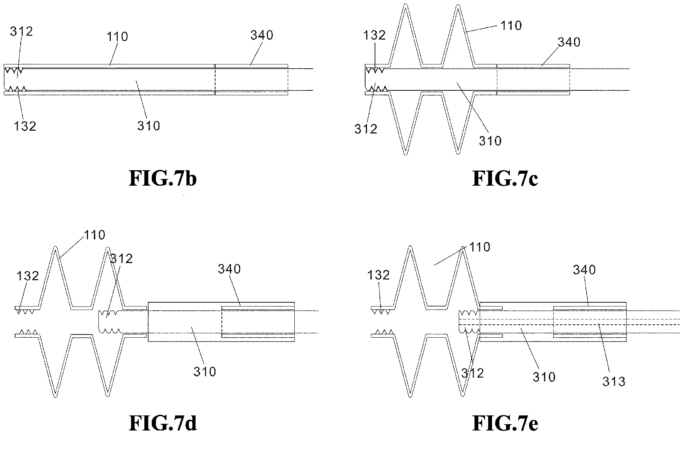

The detachable connection between the front end of the operating lever and the expanding unit can be any conventional connections (as shown in Figs. 7a, 7b, 7c & 7d), such as engaging, locking and thread engaging. The operating lever can be detached and removed from the expanding unit with such detachable connection.

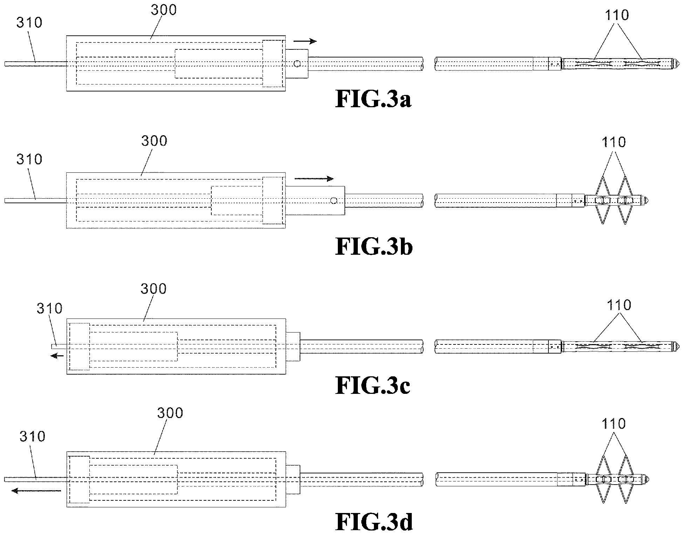

With the aforementioned configuration of the auxiliary expanding unit, operating lever and expanding unit, the device for bone fixation provided by the present invention is configured in such way that the expanding unit is expanded by pushing the auxiliary expanding unit toward the expanding unit (Figs. 3a & 3b). Alternatively, the device for bone fixation provided by the present invention can also be configured in such way that the expanding unit is expanded by pulling the auxiliary expanding unit away from the expanding unit (Figs. 3c & 3d).

The aforementioned operating lever can further be a hollowed operating lever. After the expanding unit is expanded to the expanded state by the hollowed operating lever, the hollowed operating lever can be detached from the expanding unit but not completely removed from the expanding unit, so the medical filler can be injected into the enclosing unit via the hollowed operating lever (refer to Fig. 7e).

The aforementioned injection tool is used after the auxiliary expanding unit and the operating lever have been detached, and is connected with the hollow joining tube for the injection of the medical filler.

Alternatively, the aforementioned injection tool may also be used after the auxiliary expanding unit is detached, and is connected with the hollowed operating lever for the injection of the medical filler.

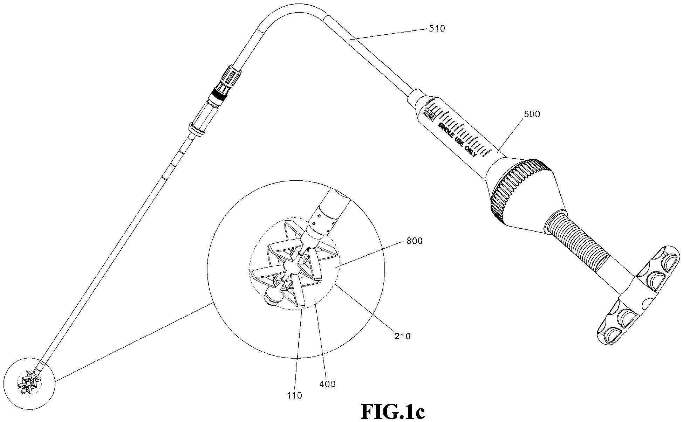

Referring to Fig. 1c, the aforementioned device for bone fixation can further include an extension tube. The extension tube is used in conjunction with the injection tool. The two ends of the extension tube can be connected with the rear end of the hollow joining tube and the injection tool respectively. The connection between the extension tube, the hollow joining tube and the injection tool can be any conventional connecting methods, such as engaging, locking and thread engaging.

The aforementioned extension tube can be any conventional extension tube that can achieve the same effect.

The aforementioned injection tool can be any conventional injection tool that can achieve the same effect.

The aforementioned device for bone fixation can further include a connecting unit, such as an engaging groove, spinal rod, bone hook, bone plate or a combination of the above, as shown in Figs. 4a, 4b, 4c, 4d, 4e and 15 of Taiwan Patent Application No. 201039796.

A method for performing bone fixation surgery provided by the present invention includes the following steps: placing an expanding unit in a contracted state into a vertebra, wherein an enclosing unit encloses the expanding unit; expanding the expanding unit to a expanded state inside the vertebra, so as to prop up the enclosing unit and the vertebra; injecting the medical filler into the enclosing unit when the enclosing unit is propped up; and, leaving the enclosing unit and the expanding unit filled with the medical filler inside the vertebra.

The expanding unit, enclosing unit and medical filler herein are the same as those described above, therefore, the detailed description thereof will be omitted hereafter.

The method for performing surgery with the bone fixation device can further include an auxiliary expanding unit, a hollow joining tube, an operating lever and an injection tool.

The aforementioned device for bone fixation can further include an extension tube.

The auxiliary expanding unit, hollow joining tube, operating lever and injection tool herein are the same as those described above, therefore, the detailed description thereof will be omitted hereafter.

According to another embodiment, the present invention provides a device for bone fixation which includes: an expanding unit, an enclosing unit, an auxiliary expanding unit, a hollow fixing unit, a pressing unit, an operating lever and an injection tool. The expanding unit has a first end and a second end, and includes two or more expanding structures capable of switching between an expanded state and a contracted state. The expanding unit includes a first expanding structure located near the first end thereof and a second expanding structure located near the second end thereof. The enclosing unit encloses the expanding unit and has a first end and a second end. The first end of the enclosing unit is secured at the first end of the expanding unit, and the second end of the enclosing unit is secured at the second end of the expanding unit. When the expanding structures of the expanding unit are switched to the expanding state, the expanding unit props up the enclosing unit. The hollow fixing unit has an injecting end and a connecting end opposite to the injecting end. The connecting end of the hollow fixing unit is detachably joined with the second end of the expanding unit. The pressing unit is joined with the injecting end of the hollow fixing unit. The auxiliary expanding unit has a coupling end and an operating end opposite to the coupling end. The coupling end of the auxiliary expanding unit is detachably coupled to the injecting end of the hollow fixing unit. The operating lever has a front end and a rear end. The front end of the operating lever is detachably joined with the expanding unit. The rear end of the operating lever is joined with the operating end of the auxiliary expanding unit, thereby allowing the auxiliary expanding unit to adjust the expanding unit to the expanded state or the contracted state by operating the operating lever. The injection tool is joined with the injecting end of the hollow fixing unit or the rear end of the operating lever, so as to inject or push medical filler into the enclosing unit via the hollow fixing unit or via the operating lever. The auxiliary expanding unit is detached and the injection tool is attached instead after the auxiliary expanding unit expands the expanding unit with the operating lever for filling the medical filler, when the filling is finished, the pressing unit is set up to press the device for bone fixation toward a bone at a surgical site.

The expanding unit, enclosing unit, medical filler and operating lever herein are the same as those described above, therefore, the detailed description thereof will be omitted hereafter.

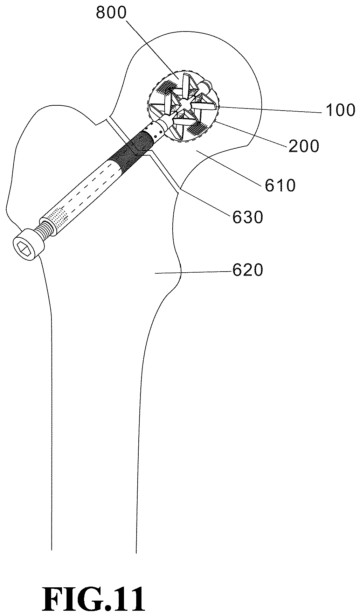

The aforementioned pressing unit can be any conventional pressing unit (see Fig. 11). After the medical filler is injected, the device for bone fixation is pressed toward the surgical site by the pressing unit, thereby reinforcing the fixing capability of the device.

The connection between the injecting end of the hollow fixing unit and the pressing unit can be any conventional connection, such as engaging, locking or thread engaging.

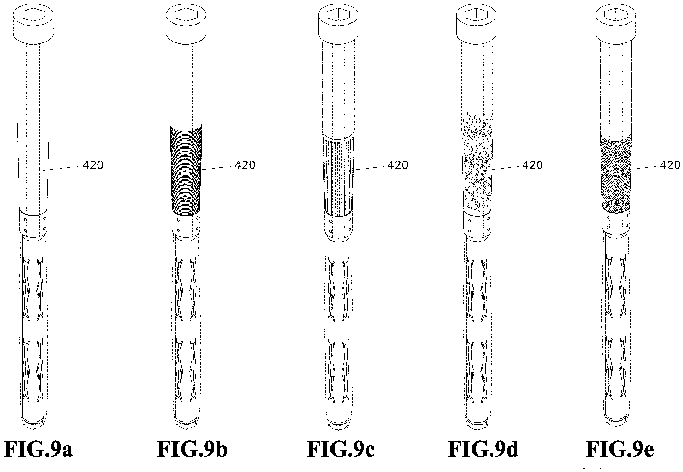

The surface between the connecting end and the injecting end of the aforementioned hollow fixing unit can have various patterns of embossments, such as ring-shaped embossments, stripe-shaped embossments, spot-shaped embossments, or mesh-shaped embossments (Figs. 9b, 9c, 9d & 9e). The embossment can increase the friction between the hollow fixing unit and the bones, so the hollow fixing unit is less likely to slide after the surgery. The body between the connecting end and the injecting end of the aforementioned hollow fixing unit can also be provided with holes, so the medical filler can flow out from the holes to bind with the bones. Alternatively, the bone cell may also grow into the holes to bind with the medical filler, thus increasing the stability between the hollow fixing unit and the bones.

The connection between the connecting end of the aforementioned fixing unit and the second end of the expanding unit can be any conventional connection, such as riveting, engaging, using retainer rings, thread engaging or locking.

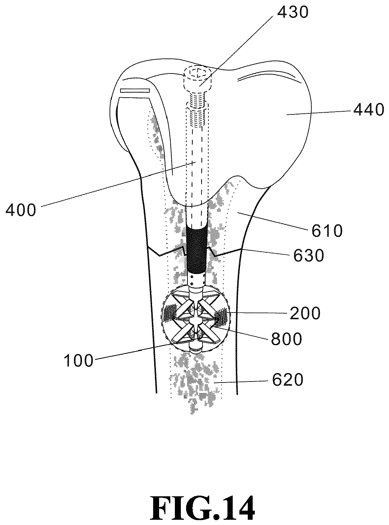

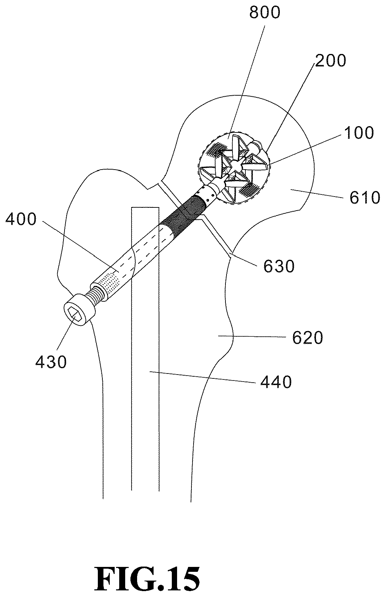

The aforementioned device for bone fixation can further include an auxiliary fixing unit (as shown in Figs. 14 and 15) to increase the stability of the bone fixation device. The auxiliary fixing unit can be a bone plate, connecting rods, bone screws, screws, rods or bone hooks.

The connection between the pressing unit and the auxiliary connecting unit can be any conventional connecting unit, such as joint replacement-screw connection (as shown in Fig. 14), bone plate-bone screw connection or bone plate-screw connection (as shown in Fig. 7a & 7b in Taiwan Patent Application No. 201112996).

The aforementioned auxiliary expanding unit adjusts the expanding structures of the expanding unit to the expanded state or the contracted state with the connection between the auxiliary expanding unit and the operating lever along with the connection between the operating lever and the expanding unit. When the expanding structures are expanded, the auxiliary expanding unit can be detached with the detachable connection between the auxiliary expanding unit and the hollow joining tube. Subsequently, the injection tool is attached for injecting the medical filler.

The second end of the aforementioned is detachably attached to the auxiliary expanding unit with any conventional detachably attachment, such as engaging, locking or thread engaging.

The aforementioned injection tool is used after the auxiliary expanding unit and the operating lever have been detached, and is connected with the hollow joining tube for the injection of the medical filler.

Alternatively, the aforementioned injection tool may also be used after the auxiliary expanding unit is detached, and is connected with the hollowed operating lever for the injection of the medical filler.

The aforementioned device for bone fixation can further include an extension tube. The extension tube is used in conjunction with the injection tool. The two ends of the extension tube can be connected with the rear end of the hollow joining tube and the injection tool respectively. The connection between the extension tube, the hollow joining tube and the injection tool can be any conventional connecting methods, such as engaging, locking and thread engaging.

The aforementioned extension tube can be any conventional extension tube that can achieve the same effect.

The aforementioned injection tool can be any conventional injection tool that can achieve the same effect.

A method for performing bone fixation surgery provided by the present invention includes the following steps: placing an expanding unit in a contracted state and a hollow fixing unit into a vertebra, wherein an enclosing unit encloses the expanding unit; expanding the expanding unit to a expanded state inside the vertebra, so as to prop up the enclosing unit and the vertebra; injecting the medical filler into the enclosing unit when the enclosing unit is propped up; leaving the enclosing unit and the expanding unit filled with the medical filler and the hollow fixing unit inside the vertebra; and, pressing the hollow fixing unit toward the bones at a surgical site with a pressing unit.

Herein, the expanding unit, enclosing unit, medical filler, hollow fixing unit and pressing unit are the same as those previously described, thus the detailed description thereof will be omitted hereafter.

The aforementioned method for performing bone fixation surgery can further include an auxiliary expanding unit, an operating lever and an injection tool. Herein, the auxiliary expanding unit, operating lever and injection tool are the same as those previously described, thus the detailed description thereof will be omitted hereafter.

### BRIEF DESCRIPTION OF THE DRAWINGS

The present invention will be apparent to those skilled in the art by reading the following detailed description of a preferred embodiment thereof, with reference to the attached drawings, in which:
Fig. 1a is a perspective view of a device for bone fixation according to a preferred embodiment of the present invention;
Fig. 1b is a perspective view of the device for bone fixation according to the preferred embodiment of the present invention in an expanded state;
Fig. 1c is a perspective view of the device for bone fixation according to the preferred embodiment of the present invention being used for injecting;
Figs. 2a, 2b, 2c, 2d, 2e, 2f, 2g, 2h, 2i, 2j are perspective views showing a expanding unit of the device for bone fixation according to the preferred embodiment of the present invention;
Figs. 3a, 3b, 3c, 3d are perspective views showing two ways for expanding the device for bone fixation according to the preferred embodiment of the present invention;
Figs. 4a, 4b, 4c, 4d are perspective views showing four ways to connect a second end of the expanding unit with a second end of an enclosing unit according to the preferred embodiment of the present invention;
Figs. 5a, 5b, 5c, 5d are perspective views showing two ways for connecting a first end of the expanding unit with a first end of the enclosing unit of the present invention;
Figs. 6a, 6b are perspective views showing two types of connection between the expanding unit and an auxiliary expanding unit according to the preferred embodiment of the present invention;
Figs. 7a, 7b, 7c, 7d, 7e are perspective views showing two types of connection between the expanding unit, auxiliary expanding unit and an operating lever according to the preferred embodiment of the present invention;
Fig. 8a is a perspective view showing a device for bone fixation according to another embodiment of the present invention;
Fig. 8b is a perspective view showing the device for bone fixation in a expanded state according to another embodiment of the present invention;
Fig. 8c is a perspective view showing the device for bone fixation according to another embodiment of the present invention after the injection process is completed;
Figs. 9a, 9b, 9c, 9d, 9e are perspective views showing five examples of the embossments on the body of a hollow fixing unit of the device for bone fixation according to another embodiment of the present invention;
Figs. 10a, 10b are perspective views showing two ways for connecting the hollow fixing unit and a pressing unit according to another embodiment of the present invention;
Fig. 11 is a perspective view illustrating the device for bone fixation according to the present invention applied to a hip joint;
Fig. 12 is a perspective view illustrating the device for bone fixation according to the present invention applied to a long bone;
Fig. 13 is another perspective illustrating the device for bone fixation according to the present invention applied to a long bone;
Fig. 14 is a perspective view illustrating the device for bone fixation according to the present invention with an auxiliary fixing unit applied to a long bone;
Fig. 15 is a perspective view illustrating the device for bone fixation according to the present invention with the auxiliary fixing unit applied to a hip joint;
Figs. 16a∼16k are perspective views illustrating surgery steps using the device for bone fixation according to the preferred embodiment of the present invention; and
Figs. 17a, 17b, 17c, 17d are perspective views illustrating surgery steps using the device for bone fixation according to another embodiment of the present invention.

### DETAILED DESCRIPTION OF THE PREFERRED EMBODIMENT

The accompanying drawings are included to provide a further understanding of the invention, and are incorporated in and constitute a part of this specification. The drawings illustrate embodiments of the invention and, together with the description, serve to explain the principles of the invention.

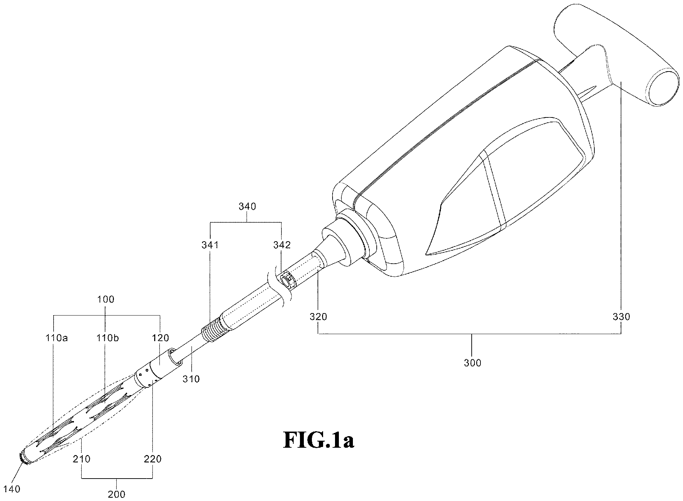

Fig. 1a is a perspective view showing a device for bone fixation according to a preferred embodiment of the present invention. As shown in Fig. 1a, expanding structures of an expanding unit includes a first expanding structure 110a and a second expanding structure 110b, which are in a contracted state in Fig. 1a. An enclosing unit 200 encloses the expanding structures 110a and 110b of the expanding unit 100 with a sphere-like enclosing member 210; meanwhile, the enclosing unit 200 is also capable of blocking off bone fragments in a vertebra to prevent the expanding unit 100 from being jammed and thus resulting in incapability of contracting to the contracted state. A second end 220 of the enclosing unit 200 is secured to the expanding unit 100 with rivets. A second end 120 of the expanding unit 100 and a front end 341 of a hollow joining tube 340 are provided with screw threads to detachably join with each other. A joining end 320 of an auxiliary expanding unit 300 is engaged with a rear end 342 of the hollowing joining tube 340 to form a detachable connection, so the auxiliary expanding unit 300 can be switched to an injection tool for injecting medical filler later. An end of an operating lever 310 forms a detachable connection with the first end of the expanding unit 100, and another end of the operating lever is connected with an operating end 330 of the auxiliary expanding unit 300. In this way, the operating end 330 of the auxiliary expanding unit 300 can be operated to move the operating lever 310, so the operating lever 310 can control the expanding or contracting of the expanding structures 110a and 110b. A fitting piece 140 is employed to reinforce the connection between the enclosing unit 200 and the expanding unit 100.

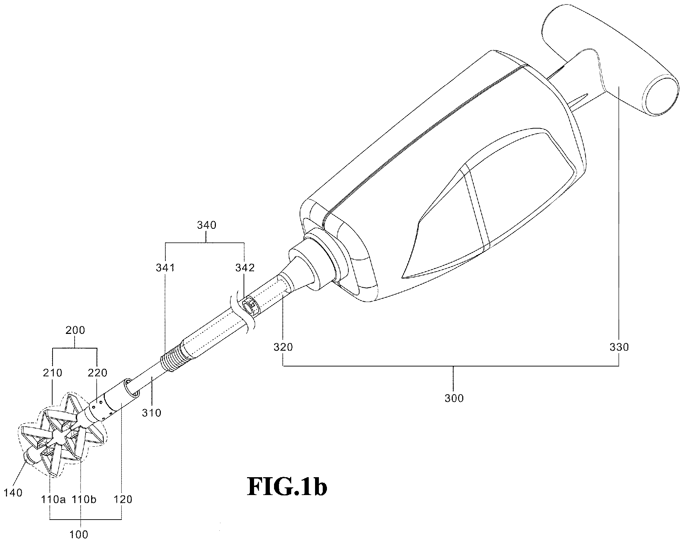

Fig. 1b is a perspective view showing the expanding unit 100 from Fig. 1a in the expanded state.

Fig. 1c is a perspective view of the device for bone fixation according to the preferred embodiment of the present invention being used for injecting medical fillers. As shown in Fig. 1c, the second end 120 of the expanding unit 100 is connected with an extension tube 510. The connection between the expanding unit 100 and the extension tube 510 can be detachable connection. When the injection of the medical filler 500 via the injection tool 500 and the extension tube 510 is completed, the extension tube 510 and the injection tool 500 can be detached.

Figs. 2a, 2b, 2c, 2d, 2e, 2f, 2g, 2h, 2i, 2j are perspective views showing the expanding unit 100 of the device for bone fixation according to the preferred embodiment of the present invention. As shown in the figures, the expanding unit 100 has a hollowed structure. Each of the expanding structures 110a and 110b includes at least four grooves 111. The grooves 111 are circularly disposed on the expanding unit 100 about a central axis thereof, so as to form a rib 112 between every two groove 111. Each of the ribs 112 at least includes a bendable joint 113. Herein, the expanding unit 100 includes the first expanding structure 110a and the second expanding structure 110b. The first expanding structure 110a is located on the expanding unit 100 close the first end thereof, and the second expanding structure 110b is located on the expanding unit 100 close to the second end 120 thereof. Each of the expanding structures 110a and 110b includes a rib length L₁ and L₂, respectively.

Figs. 2a and 2b illustrate a configuration in which the first expanding structure 110a and the second expanding structure 110b have the same rib length L₁ and L₂. Fig. 2a illustrates the expanding unit 100 in its contracted state, and Fig. 2b illustrates the expanding unit 100 in its expanded state. As shown in Figs. 2a and 2b, when the first expanding structure 110a and the second expanding structure 110b have the same rib lengths, the first expanding structure 110a and the second expanding structure 110b also have the same width in the radial direction in the expanded state.

Figs. 2c and 2d illustrate a configuration in which the rib length L₁ of first expanding structure 110a is larger than the rib length L₂ of the second expanding structure 110b. Fig. 2c illustrates the expanding unit 100 in its contracted state, and Fig. 2d illustrates the expanding unit 100 in its expanded state. As shown in Figs. 2c and 2d, when the rib length L₁ of first expanding structure 110a is larger than the rib length L₂ of the second expanding structure 110b, the first expanding structure 110a also has a width wider than the width of the second expanding structure 110b in the radial direction in the expanded state.

Figs. 2e and 2f illustrate a configuration in which the rib length L₁ of first expanding structure 110a is smaller than the rib length L₂ of the second expanding structure 110b. Fig. 2e illustrates the expanding unit 100 in its contracted state, and Fig. 2f illustrates the expanding unit 100 in its expanded state. As shown in Figs. 2e and 2f, when the rib length L₁ of first expanding structure 110a is smaller than the rib length L₂ of the second expanding structure 110b, the first expanding structure 110a also has a width narrower than the width of the second expanding structure 110b in the radial direction in the expanded state.

Figs. 2g and 2h illustrate a configuration in which a third expanding structure 110c is provided between the first expanding structure 110a and the second expanding structure 110b. The third expanding structure has a rib length L₃. Fig. 2g illustrates the expanding unit 100 in its contracted state, and Fig. 2h illustrates the expanding unit 100 in its expanded state. As shown in Figs. 2g and 2h, the rib length L₃ of the third expanding structure 110c is greater than the rib lengths L₁ and L₂ of the first and second expanding structures 110a & 110b; therefore, the third expanding structure 110c also has a width greater than the widths of the first and second expanding structures 110a & 110b in the radial direction in the expanded state.

Figs. 2i and 2j illustrate a configuration in which the ribs of the third expanding structure 110c are staggered from the ribs of the first and second expanding structures 110a & 110b. Fig. 2i illustrates the expanding unit 100 in its contracted state, and Fig. 2j illustrates the expanding unit 100 in its expanded state. As shown in Figs. 2i and 2j, the ribs of the third expanding structure 110c are staggered from the ribs of the first and second expanding structures 110a & 110b when viewed from the first end of the expanding unit 100 toward the second end of the expanding unit.

Figs. 3a, 3b, 3c, 3d are perspective views showing two ways for expanding the device for bone fixation according to the preferred embodiment of the present invention. As shown in Figs. 3a and 3b, the expanding structure 110 can be switched from the contracted state to the expanded state by pushing the operating lever 310 toward the direction of the expanding unit 110. As shown in Figs. 3c and 3d, the expanding structure 110 can be switched from the contracted state to the expanded state by pulling the operating lever 310 away from the expanding unit 110. Among the two ways for expanding the device for bone fixation, the pulling as shown in Figs. 3c and 3d is the preferable method.

Figs. 4a, 4b, 4c, 4d are perspective views showing four ways to connect the second end of the expanding unit with the second end of the enclosing unit according to the preferred embodiment of the present invention. Fig. 4a illustrates that the second end 120 of the expanding unit 100 is secured at the second end 220 of the enclosing unit 200 with rivets. Fig. 4b illustrates that the second end 120 of the expanding unit 100 is secured at the second end 220 of the enclosing unit 200 by locking. Fig. 4c illustrates that the second end 120 of the expanding unit 100 is secured at the second end 220 of the enclosing unit 200 with a retainer ring. Fig. 4d illustrates that the second end 120 of the expanding unit 100 is secured at the second end 220 of the enclosing unit 200 by binding. Among the connection methods mentioned above, the connection with rivets as shown in Fig. 4a is the preferred method.

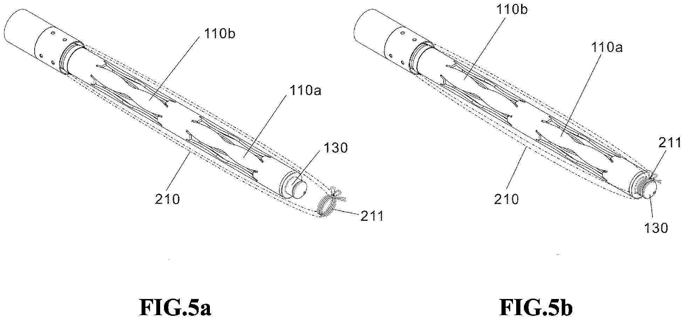

Figs. 5a, 5b, 5c, 5d are perspective views showing two ways for connecting the first end of the expanding unit with the first end of the enclosing unit of the present invention. Figs. 5a and 5b illustrate that a sphere-like double-layered enclosing member 210 is fixed to a protrusion 130 of the expanding unit by the binding of a fixing member 211. Figs. 5c and 5d illustrate that the sphere-like double-layered enclosing member 210 is fixed to the protrusion 130 of the expanding unit by a ring-shaped fixing member 211. Among the connection methods mentioned above, the ring-shaped fixing member 211 as shown in Figs. 5c and 5d is the preferred method.

Figs. 6a, 6b are perspective views showing two types of connection between the expanding unit and the auxiliary expanding unit according to the preferred embodiment of the present invention. Fig. 6a illustrates that the second end 120 of the expanding unit 100 is detachably connected with the joining end 320 of the auxiliary expanding unit 300 with thread engaging. Fig. 6b illustrates that the second end 120 of the expanding unit 100 is detachably connected with the joining end 320 of the auxiliary expanding unit 300 by engaging. Among the two connection methods mentioned above, the thread engaging connection as shown in Fig. 6a is the preferred method.

Fig. 7a, 7b, 7c, 7d, 7e are perspective views showing two types of connection between the expanding unit, auxiliary expanding unit and the operating lever according to the preferred embodiment of the present invention. Fig. 7a illustrates that an engaging groove 131 is provided at the first end of the expanding unit, and an engaging protrusion 311 is provided on the operating lever 310. The engaging groove 131 forms detachable connection with the engaging protrusion 311, so the operating lever 310 can be detached and the injection tool can be attached instead when the operating lever 310 is done expanding the expanding unit 100. The fixing protrusion 321 on the body 340 of the auxiliary expanding unit 300 also forms a detachable connection with the fixing groove 121 on the expanding unit 100 by engaging with each other. Figs. 7b∼7e illustrate that the operating lever 310 can be operated to expand the expanding structure 110 with the engagement between the upper thread 312 and the thread 132 on the inner circumferential surface at the first end of the expanding unit 100. When the expansion is completed, the operating lever 310 can be pulled out from the expanding structure 110. Fig. 7e illustrates that the operating lever 310 is provided with a hollowed passage 313, which can be used to inject medical filler after the operating lever 310 has been removed.

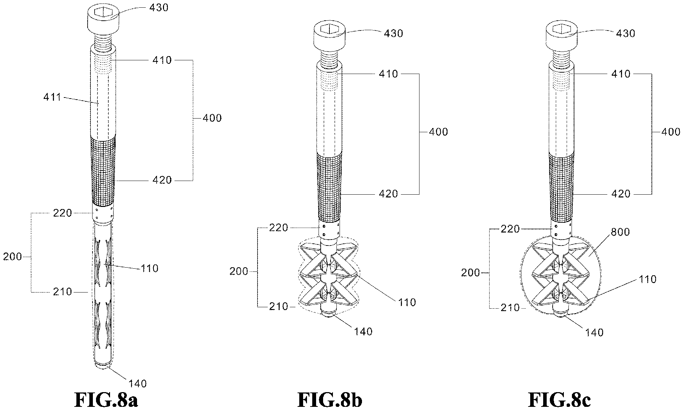

Fig. 8a is a perspective view showing a device for bone fixation according to another embodiment of the present invention. As shown in Fig. 8a, a hollow fixing unit 400 is connected with the expanding unit 100 with rivets. The body 420 of the hollow fixing unit 400 is provided with embossment for increasing the friction between the hollow fixing unit 400 and the bones, so the hollow fixing unit 400 is less likely to slide after surgery. In addition, the hollow fixing unit 400 is provided with an injection passage 411 for injecting medical filler. When the injecting is completed, a pressing unit 430 can be installed to press the expanding unit toward the bones at the surgical site.

Fig. 8b is a perspective view showing the expanding structure 110 from Fig. 8a in the expanded state.

Fig. 8c is a perspective view showing the expanding structure 110 from Fig. 8b after the injection of medical filler is completed.

Fig. 9a, 9b, 9c, 9d, 9e are perspective views showing five examples of the embossments on the body of the hollow fixing unit of the device for bone fixation according to another embodiment of the present invention. Fig. 9a illustrates that the body 420 of the hollow fixing device is provided without any embossments. Fig. 9b illustrates that the body 420 of the hollow fixing device has ring-shaped embossments. Fig. 9c illustrates that the body 420 of the hollow fixing device stripe-shaped embossments. Fig. 9d illustrates that the body 420 of the hollow fixing device has spotted embossments. Fig. 9e illustrates that the body 420 of the hollow fixing device has mesh-shaped embossments. Among the above mentioned embossments, the stripe-shaped embossments and the spotted embossments as shown in Fig. 9b and Fig. 9d respectively are the preferred choices.

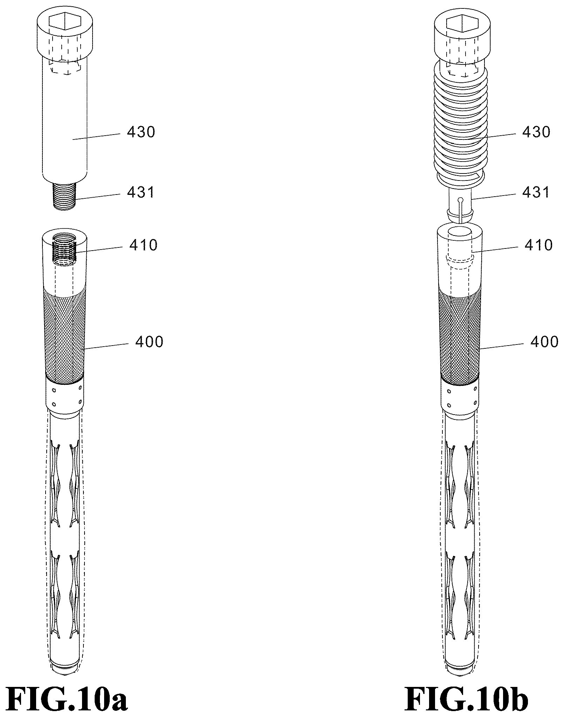

Fig. 10a and 10b are perspective views showing two ways for connecting the hollow fixing unit and the pressing unit according to another embodiment of the present invention. Fig. 10a illustrates that the connecting end 431 of the pressing unit 430 is connected to the injecting end 410 of the hollow fixing unit 400 by thread engaging. Fig. 10b illustrates that the connecting end 431 of the pressing unit 430 is fitted into the injecting end 410 of the hollow fixing unit 400.

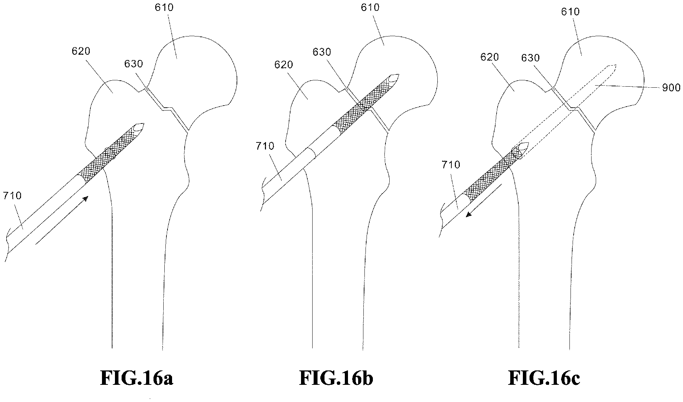

Fig. 11 is a perspective view illustrating the device for bone fixation according to the present invention applied to a hip joint. After the hollow fixing unit 400, the expanding unit 100 and the enclosing unit 200 pass through a rear broken bone 620, a crack 630 and a front broken bone 610, the medical filler 800 is injected to connect the front broken bone 610 with the rear broken bone 620. Subsequently, the pressing unit 430 is used to further tighten the connection between the front broken bone 610, rear broken bone 620 and hollow fixing unit 400, thereby increasing the stability thereof. Steps of performing bone fixation surgery are shown in Figs. 16a∼16k.

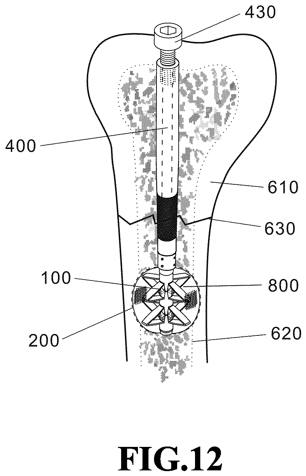

Fig. 12 is a perspective view illustrating the device for bone fixation according to the present invention applied to a long bone. After the hollow fixing unit 400, the expanding unit 100 and the enclosing unit 200 pass through a rear broken bone 620, a crack 630 and a front broken bone 610, the medical filler 800 is injected to connect the front broken bone 610 with the rear broken bone 620. Subsequently, the pressing unit 430 is used to further tighten the connection between the front broken bone 610, rear broken bone 620 and hollow fixing unit 400, thereby increasing the stability thereof. Steps of performing bone fixation surgery are shown in Figs. 16a∼16k.

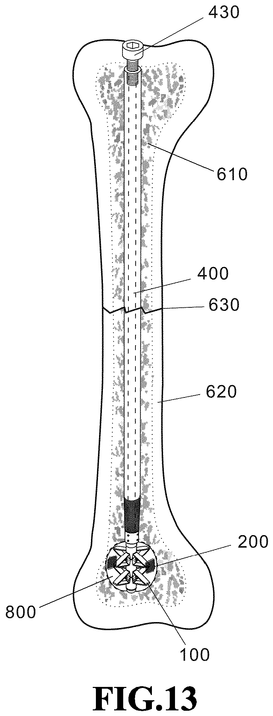

Fig. 13 is another perspective illustrating the device for bone fixation according to the present invention applied to a long bone. After the hollow fixing unit 400, the expanding unit 100 and the enclosing unit 200 pass through a rear broken bone 620, a crack 630 and a front broken bone 610, the medical filler 800 is injected to connect the front broken bone 610 with the rear broken bone 620. Subsequently, the pressing unit 430 is used to further tighten the connection between the front broken bone 610, rear broken bone 620 and hollow fixing unit 400, thereby increasing the stability thereof. Steps of performing bone fixation surgery are shown in Figs. 16a∼16k.

Fig. 14 is a perspective view illustrating the device for bone fixation according to the present invention with an auxiliary fixing unit applied to a long bone. After the hollow fixing unit 400, the expanding unit 100 and the enclosing unit 200 pass through a rear broken bone 620, a crack 630, a front broken bone 610 and an auxiliary fixing unit 440, the medical filler 800 is injected to connect the front broken bone 610 with the rear broken bone 620. Subsequently, the pressing unit 430 is used to further tighten the connection between the front broken bone 610, rear broken bone 620, hollow fixing unit 400 and the auxiliary fixing unit 440, thereby increasing the stability thereof. Steps of performing bone fixation surgery are shown in Figs. 16a∼16k.

Fig. 15 is a perspective view illustrating the device for bone fixation according to the present invention with the auxiliary fixing unit applied to a hip joint. After the hollow fixing unit 400, the expanding unit 100 and the enclosing unit 200 pass through a rear broken bone 620, a crack 630, a front broken bone 610 and an auxiliary fixing unit 440, the medical filler 800 is injected to connect the front broken bone 610 with the rear broken bone 620. Subsequently, the pressing unit 430 is used to further tighten the connection between the front broken bone 610, rear broken bone 620, hollow fixing unit 400 and the auxiliary fixing unit 440, thereby increasing the stability thereof. Steps of performing bone fixation surgery are shown in Figs. 16a∼16k.

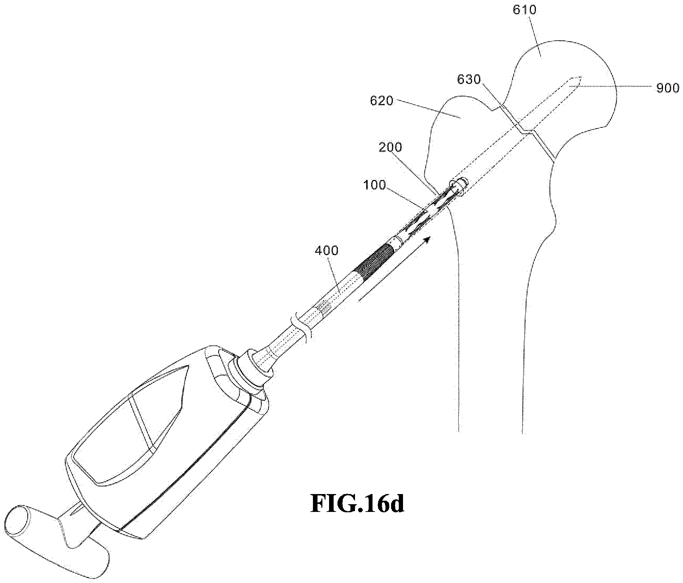

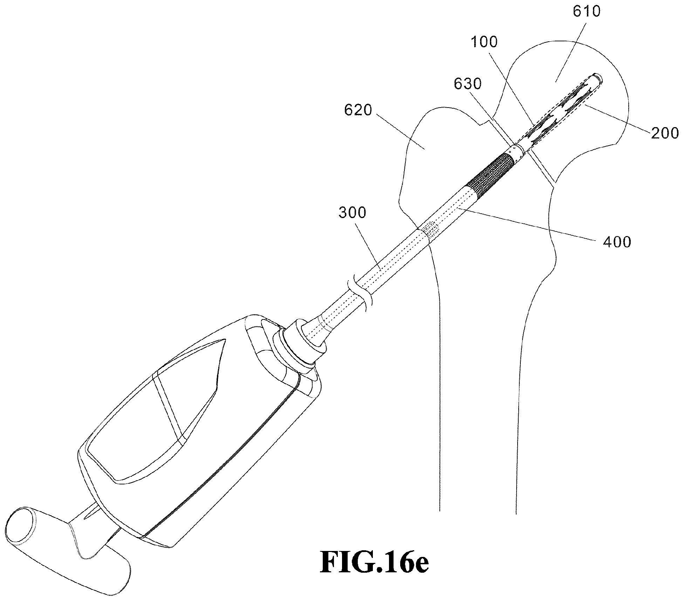

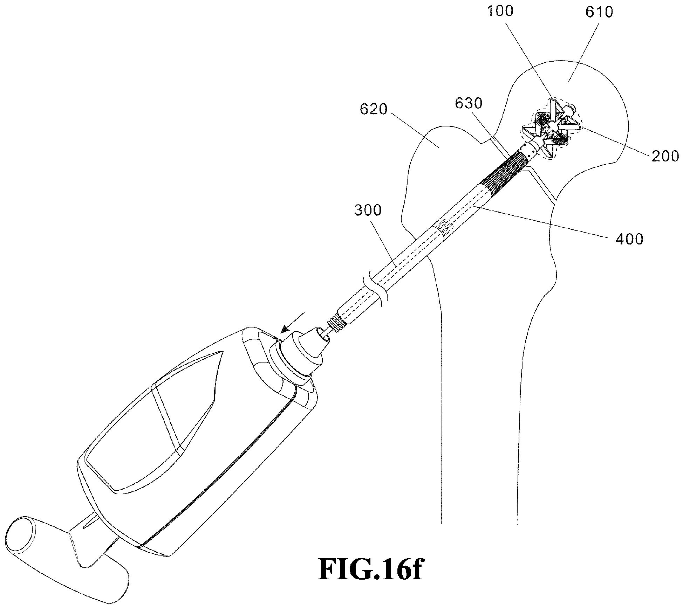

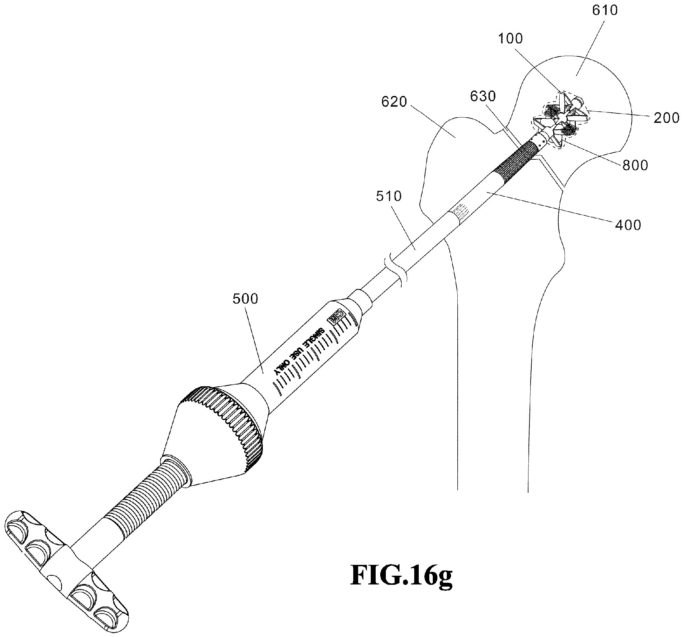

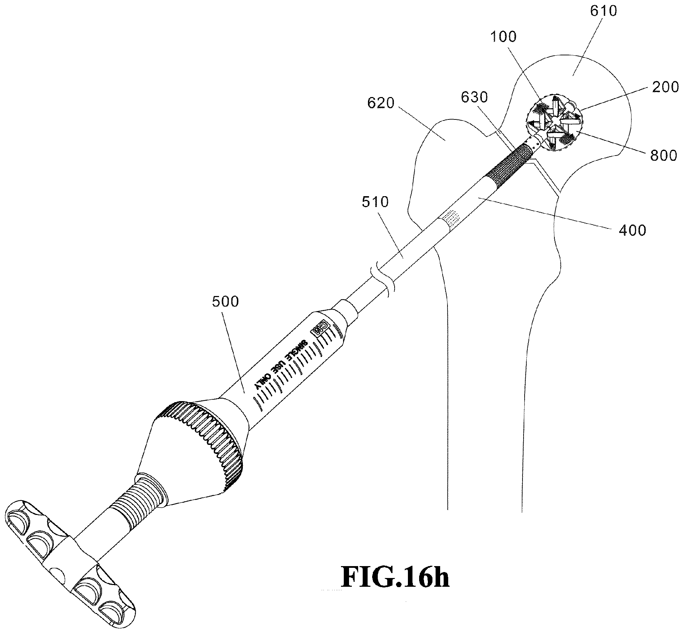

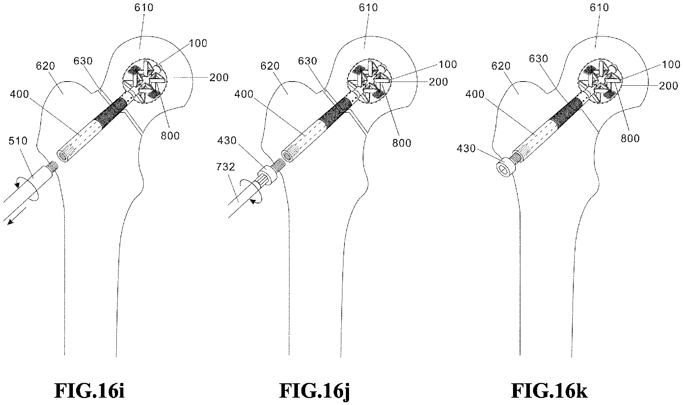

Figs. 16a∼16k are perspective views illustrating surgery steps using the device for bone fixation according to the preferred embodiment of the present invention. Fig. 16a illustrates that a drill 710 drills into a rear broken bone 620. Fig. 16b illustrates that the drill 710 passes through the rear broken bone 620 and a crack 630 and enters a front broken bone 610. Fig. 16c illustrates that a cavity 900 is generated after the drill 710 passes through the rear broken bone 620 and a crack 630 and enters a front broken bone 610. Fig. 16d illustrates that the hollow fixing unit 400, the enclosing unit 200 and the expanding unit 100 are being placed in the cavity 900. Fig. 16e illustrates that the hollow fixing unit 400, enclosing unit 200 and expanding unit 100 is completely placed inside the cavity 900. Fig. 16f illustrates that the expanding unit 100 is adjusted to the expanded state by the auxiliary expanding unit 300. Fig. 16g illustrates that the auxiliary expanding unit 300 has been replaced by the injection tool 500 and the extension tube 510 for injecting the medical filler 800. Fig. 16h illustrates that the injection of the medical filler 800 is completed. Fig. 16i illustrates that the extension tube 510 is being removed from the hollow fixing unit 400. Fig. 16j illustrates that the pressing unit 430 is being locked into the hollow fixing unit 400 by a locking device 732. Fig. 16k illustrates that the pressing unit 430 is locked into the hollow fixing unit 400, and thus tightening the connection between the front broken bone 610, rear broken bone 620 and hollow fixing unit 400.

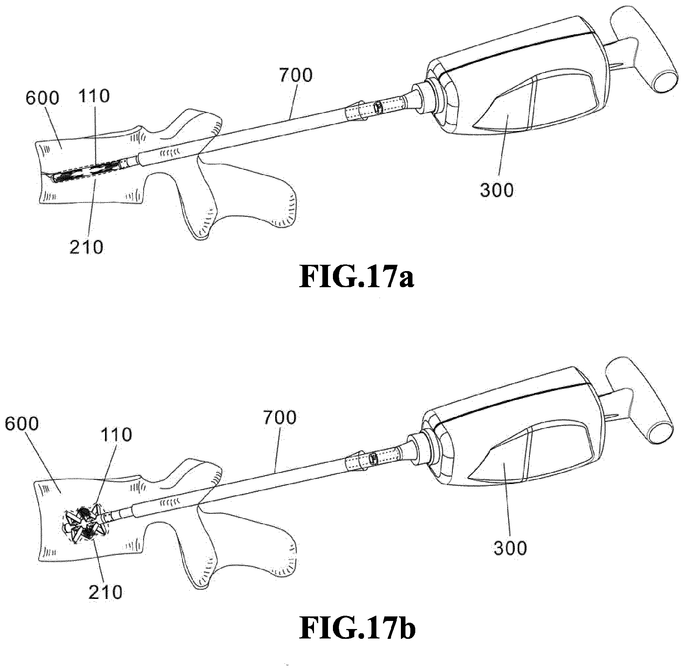

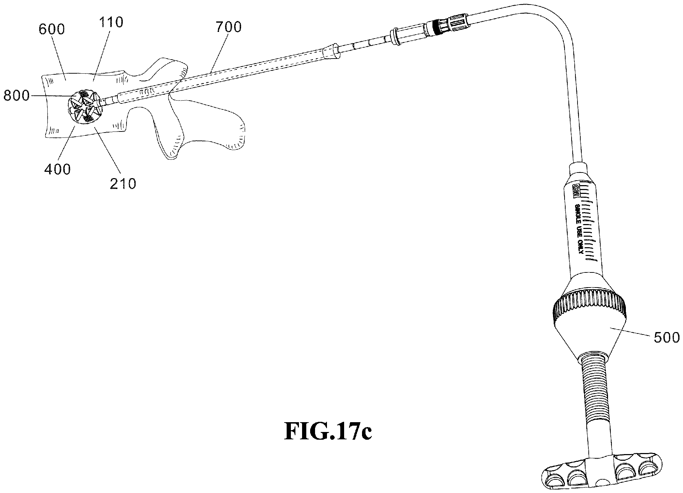

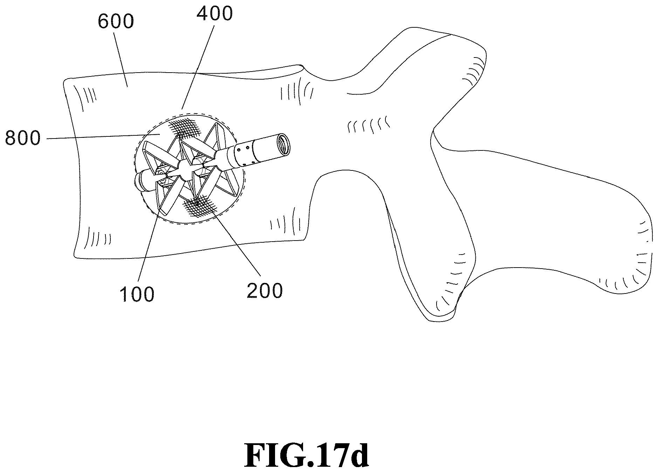

Figs. 17a, 17b, 17c, 17d are perspective views illustrating surgery steps using the device for bone fixation according to another embodiment of the present invention. Fig. 17a illustrates that the enclosing unit 200 and the expanding unit 100 are placed into a vertebra 600 via a tube 700. Fig. 17b illustrates that the expanding structures 110 are expanded by the auxiliary expanding unit 300, and the sphere-like enclosing member 210 is propped up by the expanding structures 110. Fig. 17c illustrates that the auxiliary expanding unit 300 is replaced by extension tube 510 and the injection tool 500 for injecting the medical filler 800. Fig. 17d illustrates that after the injection of the medical filler 800 is completed, the device for bone fixation props up the vertebra 600 effectively.

Although the present invention has been described with reference to the preferred embodiments thereof, it is apparent to those skilled in the art that a variety of modifications and changes may be made without departing from the scope of the present invention which is intended to be defined by the appended claims.

## Claims

1. A device for bone fixation, comprising:
an expanding unit (100) having a first end and a second end (120), and including two or more expanding structures capable of switching between an expanded state and a contracted state, wherein the expanding unit includes a first expanding structure (110a) located near the first end thereof and a second expanding structure (110b) located near the second end (120) thereof; and
an enclosing unit (200), enclosing the expanding unit and having a first end and a second end (220), wherein the first end of the enclosing unit is secured at the first end of the expanding unit, and the second end of the enclosing unit is secured at the second end of the expanding unit;
**characterised in that** the enclosing unit is a mesh-shaped enclosing unit or a porous enclosing unit, and the enclosing unit is made of biocompatible materials; and
wherein, when the device for bone fixation is placed inside a bone, the expanding structures of the expanding unit can be switched to the expanding state and thereby propping up the enclosing unit, and a medical filler can be filled into the enclosing unit through the second end of the expanding unit such that a small amount or a part of the medical filler exudes from holes of the enclosing unit and binds with trabecular bones.

2. The device for bone fixation according to claim 1, wherein the expanding unit has a hollowed structure, and each said expanding structure comprises:
a plurality of grooves, disposed circularly on the expanding unit about an axis of the expanding unit, so as to form a rib between every two of the grooves;
wherein, each rib has at least one bendable joint.

3. The device for bone fixation according to claim 2, wherein one or more third expanding structures are disposed between the first expanding structure and the second expanding structure.

4. The device for bone fixation according to claim 2, wherein the ribs of the expanding structures are aligned with each other when viewed from the first end of the expanding unit toward the second end of the expanding unit.

5. The device for bone fixation according to claim 3, wherein the ribs of at least one of the expanding structures are staggered from the ribs of the rest of the expanding structures when viewed from the first end of the expanding unit toward the second end of the expanding unit.

6. The device for bone fixation according to any one of claims 2 to 5, wherein each expanding structure has a rib length, and the rib length of at least one of the expanding structures is different from the rib lengths of the rest of the expanding structures.

7. The device for bone fixation according to any one of claims 2 to 5, wherein each expanding structure has a rib length, and the rib lengths of all of the expanding structures are the same.

8. The device for bone fixation according to claim 1, wherein the second end of the enclosing unit is secured at the second end of the expanding unit with a plurality of rivets.

9. The device for bone fixation according to claim 1, wherein a protrusion is disposed at the first end of the expanding unit, a ring-shaped fixing member is disposed at the first end of the enclosing unit, and the first end of the enclosing unit is secured at the first end of the expanding unit by fixing the ring-shaped fixing member with the protrusion.

10. The device for bone fixation according to claim 1, further comprising:
a hollow joining tube, having a front end and a rear end, wherein the front end of the hollow joining tube is detachably joined with the second end of the expanding unit;
an auxiliary expanding unit, having a coupling end and an operating end opposite to the coupling end, wherein the coupling end of the auxiliary expanding unit is detachably coupled to the rear end of the hollow joining tube;
an operating lever, having a front end and a rear end, wherein the front end of the operating lever is detachably joined with the expanding unit, the rear end of the operating lever is joined with the operating end of the auxiliary expanding unit, thereby allowing the auxiliary expanding unit to adjust the expanding unit to the expanded state or the contracted state when the operating lever is operated; and
an injection tool, joined with the rear end of the hollow joining tube or the rear end of the operating lever, so as to inject or push a medical filler into the enclosing unit via the hollow joining tube or via the operating lever;
wherein the auxiliary expanding unit is detached and the injection tool is attached after the auxiliary expanding unit expands the expanding unit with the operating lever for filling the medical filler.

11. The device for bone fixation according to claim 1, further comprising:
a hollow fixing unit, having an injecting end and a connecting end opposite to the injecting end, wherein the connecting end of the hollow fixing unit is detachably joined with the second end of the expanding unit;
a pressing unit, joined with the injecting end of the hollow fixing unit;
an auxiliary expanding unit, having a coupling end and an operating end opposite to the coupling end, wherein the coupling end of the auxiliary expanding unit is detachably coupled to the injecting end of the hollow fixing unit;
an operating lever, having a front end and a rear end, wherein the front end of the operating lever is detachably joined with the expanding unit, the rear end of the operating lever is joined with the operating end of the auxiliary expanding unit, thereby allowing the auxiliary expanding unit to adjust the expanding unit to the expanded state or the contracted state when the operating lever is operated; and
an injection tool, joined with the injecting end of the hollow fixing unit or the rear end of the operating lever, so as to inject or push a medical filler into the enclosing unit via the hollow fixing unit or via the operating lever;
wherein the auxiliary expanding unit is detached and the injection tool is attached instead after the auxiliary expanding unit expands the expanding unit with the operating lever for filling the medical filler, when the filling is finished, the pressing unit is set up to press the device for bone fixation toward a bone at a surgical site.

## Patentansprüche

1. Eine Vorrichtung zum Befestigen von Knochen, die Folgendes umfasst:
Eine Spreitzeinheit (100) mit einem ersten und zweiten Ende (120), die zwei oder mehr Spreizstrukturen aufweist und die zwischen einer gespreizten Lage und einer eingezogenen Lage eingestellt werden kann,
wobei die Spreizeinheit eine erste Spreizstruktur (110a) umfasst, die in der Nähe des ersten Endes der Einheit angebracht ist und eine zweite Spreizstruktur (110b), die in der Nähe des zweiten Endes der Enheit (120) angebracht ist;
und ein Gehäuseteil (200), das die Spreizeinheit einschliesst und ein erstes und ein zweites Ende (220) aufweist, wobei das erste Ende des Gehäuseteils am ersten Ende der Spreizeinheit gesichert ist, und das zweite Ende des Gehäuseteils am zweiten Ende der Spreizeinheit befestigt ist.
**dadurch gekennzeichnet, dass**
das Gehäuseteil eine maschenartige Form oder eine poröse Form aufweist, und das Gehäuseteil aus einem biokompatiblen Material gefertigt ist; und
wobei, wenn die Vorrichtung für das Befestigen der Knochen innerhalb eines Knochens eingebaut wird, die Spreizstrukturen der Spreizeinheit auf die gespreizte Lage eingestellt werden können und so das Gehäuseteil abstüzen, und wobei eine medizinisches Füllsubstamz in das Gehäuseteil durch das zweite Ende der Spreizeinheit eingebracht werden kann, so dass eine kleine Menge oder ein Teil der medizinischen Füllsubstanz durch die Öffnungen de Gehäuseteils austreten kann und an den Schwammknochen anbindet.

2. Die Vorrichtung für das Befestigen von Knochen gemäss Anspruch 1, bei der die Spreizeinheit eine ausgehöhlte Struktur aufweist, und besagte Spreizstruktur Folgendes umfasst:
Mehrere Rillen, die kreisförmig auf der Spreizeinheit um eine Achse der Spreizeinheit verteilt sind, so dass eine Rippe zwischen je zwei der Rillen geformt wird;
Wobei jede Rippe mindestens eine biegbare Verbindung aufweist.

3. Die Vorrichtung zum Befestigen von Knochen gemäss Anspruch 2, bei der eine oder mehrere dritte Spreizstrukturen zischen der ersten und zweiten Spreizstruktur angeordnet ist/sind.

4. Die Vorrichtung zum Befestigen von Knochen gemäss Anspruch 2, bei der die Rippen der Spreizstrukturen miteinander ausgerichtet sind, gesehen vom ersten Ende der Spreizeinheit hin zum zweiten Ende der Spreizeinheit.

5. Die Vorrichtung zum Befestigen von Knochen gemäss Anspruch 3, bei der die Rippen von mindestens einer der Spreizstrukturen mit Bezug auf die Rippen der restlichen Spreizstrukturen versetzt sind, gesehen ab dem ersten Ende der Spreizeinheit hin zum zweiten Ende der Spreizeinheit.

6. Die Vorrichtung zum Befestigen von Knochen gemäss irgendeinem der Ansprüche 2 bis 5, bei der jede Spreizstruktur eine Rippenlänge hat und die Rippenlänge von mindestens einer Spreizstruktur sich von den Rippenlängen der restlichen Spreizstrukturen unterscheidet.

7. Die Vorrichtung zum Befestigen von Knochen gemäss irgendeinem der Ansprüche 2 bis 5, bei der jede Spreizstruktur eine Rippenlänge aufweist und die Rippenlänge von allen Spreizstrukturen dieselbe ist.

8. Die Vorrichtung zum Befestigen von Knochen übereinstimmend mit Anspruch 1, bei der das zweite Ende des Gehäuseteils am zweiten Ende der Spreizeinheit mit mehreren Nieten befestigt ist.

9. Die Vorrichtung zum Befestigen von Knochen übereinstimmend mit Anspruch 1, bei der am ersten Ende der Spreizeinheit ein Vorsprung vorgesehen ist, ein ringförmiges Befestigungsglied am ersten Ende des Gehäuseteils angeordenet ist, und das erste Ende des Gehäuseteils am ersten Ende der Spreizeinheit durch Befestigung des ringförmigen Befestigungsgliedes am Vorsprung gesichert wird.

10. Die Vorrichtung zum Befestigen von Knochen übereinstimmend mit Anspruch 1, die ausserdem Folgendes umfasst:
Ein hohles Verbindungsrohr mit einen vorderen und einem hinteren Ende, wobei das Vorderende des hohlen Verbindungsrohres mit dem zweiten Ende der Spreizeinheit lösbar verbunden ist.;
Eine Hilfsspreizeinheit mit einem Kupplungsende und einem Betätigungsende gegenüber dem Kupplungsende, wobei das Kupplungsende der Hilfsspreizeinheit lösbar am hinteren Ende des hohlen Verbindungsrohres angeschlossen ist;
Einen Betätigungshebel mit einem vorderen und einem hinteren Ende, wobei das Vorderende des Betätigungshebels lösbar mit der Spreizeinheit verbunden ist, das hintere Ende des Betätigungshebels mit dem Betätigungsende der Hilsspreizeinheit verbunden ist, wodurch die Hilfsspreizeinheit die Spreizeinheit auf den gespreizten Zustand oder eingezogenen Zustand einstellen kann, wenn der Betätigungshebel bedient wird; und
Ein Spritzgerät, das mit dem hinteren Ende des hohlen Verbindungsrohres oder dem hinteren Ende des Betätigungshebels verbunden ist, um eine medizinische Füllsubstanz in das Gehäuseteil durch das hohle Verbindungsrohr oder über den Betätigungshebel einzuspritzen oder einzudrücken;
wobei die Hilfsspreizeinheit abmontiert und das Spritzgerät montiert wird, nachdem die Hilfsspreizeinheit die Spreizeinheit mit dem Betätigungshebel zum Befüllen mit der medizinischen Füllsubstanz gespreizt hat.

11. Die Vorrichtung zum Befestigen von Knochen übereinstimmend mit Anspruch 1, die weiterhin Folgendes umfasst:
Eine hohle Befestigungseinheit mit einem Einspritzende und einem Anschlussende gegenüber dem Einspritzende, wobei das Anschlussende der hohlen Befestigungseinheit lösbar mit dem zweiten Ende der Spreizeinheit verbunden ist;
Eine Presseinheit, die mit dem Einspritzende der hohlen Befestigungseinheit verbunden ist;
Eine Hilsspreizeinheit mit einem Kupplungsende und einen Betätigungsende gegenüber dem Kupplungsende, wobei das Kupplungsende der Hilfsspreizeinheit lösbar mit dem Einspritzende der hohlen Befestigungseinheit verbunden ist;
Einen Betätigungshebel mit einem vorderen und einem hinteren Ende, wobei das Vorderende des Betätigungshebels lösbar mit der Spreizeinheit verbunden ist, das hintere Ende des Betätigungshebels mit dem Betätigungsende der Hilfsspreizeinheit verbunden ist, wodurch die Hilfsspreizeinheit die Spreizeinheit in den gespreizten oder eingezogenen Zustand einstellen kann, wenn der Betätigungshebel bedient wird; und
Ein Spritzgerät, das an das Einspritzende der hohlen Befestigungseinheit oder das hinteren Ende des Betätigungshebels angeschlossen ist, um so das medizinische Füllmaterial in das Gehäuseteil über die hohle Befestigungseinheit oder über den Betätigungshebel einzuspritzen oder einzuschieben,
wobei die Hilfsspreizeinheit gelöst wird und das Spritzgerät an seiner Stelle angeschlossen wird, nachdem die Hilfsspreizeinheit die Spreizeinheit mit dem Betätigungshebel für das Füllen mit der medizinischen Füllsubstanz gespreizt hat, wenn die Befüllung beendet ist, und die Presseinheit vorbereitet ist, um die Vorrichtung zum Befestigen von Knochen in einem quirurgischen Bereich zu einem Knochen hinzuschieben.

## Revendications

1. Un dispositif de fixation osseuse comprenant:
une unité extensible (100), avec une première extrémité et une deuxième extrémité (120), qui comprend deux ou plusieurs structures extensibles pouvant passer d'une condition expansée à une condition contractée, où l'unité extensible comprend une première structure extensible (110a) située à proximité de la première extrémité et une seconde structure extensible (110b) située à proximité de la deuxième extrémité (120) correspondante ; et
une unité enveloppante (200), renfermant l'unité extensible, dotée d'une première extrémité et d'une seconde extrémité (220),
où la première extrémité de l'unité enveloppante est fixée à la première extrémité de l'unité extensible, et la deuxième extrémité de l'unité enveloppante est fixée à la deuxième extrémité de l'unité extensible ;
étant **caractérisée par le fait que** l'unité enveloppante est en forme de maille ou poreuse, et elle est composée de matériaux biocompatibles ; et
où, lorsque le dispositif de fixation osseuse est situé à l'intérieur d'un os, les structures extensibles de l'unité extensible peuvent passer à la condition extensible et, par conséquent, elles étaient l'unité enveloppante, et un remplisseur médical peut être rempli à l'intérieur de l'unité enveloppante à travers de la deuxième l'extrémité de l'unité extensible, de telle manière qu'une petite quantité ou une partie du remplisseur médical exsude des orifices de l'unité enveloppante et se lie à l'os trabéculaire.

2. Le dispositif de fixation osseuse conformément à la Revendication 1, où l'unité extensible a une structure en creux, et chacune desdites structures extensibles comprend :
une pluralité de rainures, situées de manière circulaire sur l'unité extensible autour d'un axe de celle-ci afin de former une nervure entre deux rainures ;
où chaque nervure a au moins un joint pliable.

3. Le dispositif de fixation osseuse conformément à la Revendication 2, où une ou plusieurs troisièmes structures extensibles sont situées entre la première structure extensible et la deuxième structure extensible.

4. Le dispositif de fixation osseuse conformément à la Revendication 2, où les nervures des structures extensibles sont alignées entre elles, lorsqu'elles sont vues depuis la première extrémité de l'unité extensible vers la deuxième extrémité de cette unité.

5. Le dispositif de fixation osseuse conformément à la Revendication 3, où les nervures d'au moins l'une des structures extensibles est décalée par rapport aux nervures du reste des structures extensibles, lorsqu'elles sont vues depuis la première extrémité de l'unité extensible vers la deuxième extrémité de cette unité.

6. Le dispositif de fixation osseuse conformément à l'une quelconque des Revendications 2 à 5, où chaque structure extensible a une longueur de nervure, et la longueur de la nervure d'au moins l'une des structures extensibles est différente des longueurs des nervures du reste des structures extensibles.

7. Le dispositif de fixation osseuse conformément à l'une quelconque des Revendications 2 à 5, où chaque structure extensible a une longueur de nervure, et les longueurs des nervures de toutes les structures extensibles sont égales.

8. Le dispositif de fixation osseuse conformément à la Revendication 1, où la deuxième extrémité de l'unité enveloppante est fixée à la deuxième extrémité de l'unité extensible avec plusieurs rivets.

9. Le dispositif de fixation osseuse conformément à la Revendication 1, où une saillie est située sur la première extrémité de l'unité extensible ; une pièce de fixation en forme d'anneau est placée à la première extrémité de l'unité enveloppante ; et la première extrémité de l'unité enveloppante est fixée à la première extrémité de l'unité extensible au moyen de la fixation de la pièce en forme d'anneau à la saillie.

10. Le dispositif de fixation osseuse conformément à la Revendication 1 comprenant en outre :
un tube de raccordement creux, avec une extrémité avant et une extrémité arrière, où l'extrémité avant du tube de raccordement creux est assemblée de manière détachable à la deuxième extrémité de l'unité extensible ;
une unité extensible auxiliaire, qui comprend une extrémité de couplage et une extrémité de manoeuvre en face de l'extrémité de couplage, où l'extrémité de couplage de l'unité extensible auxiliaire est assemblée de manière détachable à l'extrémité arrière du tube de raccordement creux ;
un levier de manoeuvre, qui comprend une extrémité avant et une extrémité arrière, où l'extrémité avant du levier de manoeuvre est assemblée de manière détachable à l'unité extensible ; l'extrémité arrière du levier de manoeuvre est assemblée à l'extrémité de manoeuvre de l'unité extensible auxiliaire, et, par conséquent, elle permet que l'unité extensible auxiliaire puisse ajuster l'unité extensible conformément à la condition d'expansé ou de contracté lorsque le levier de manoeuvre est en fonctionnement ; et
un outillage d'injection, assemblé à l'extrémité arrière du tube de raccordement creux ou à l'extrémité arrière du levier de manoeuvre, de façon à injecter ou pousser un remplisseur médical dans l'unité enveloppante à travers du tube de raccordement creux ou à travers du levier de manoeuvre ;
où l'unité extensible auxiliaire est détachée, et l'outillage d'injection est rattaché après que l'unité extensible auxiliaire ait étendu l'unité extensible au moyen du levier de manoeuvre pour remplir le remplisseur médical.

11. Le dispositif de fixation osseuse conformément à la Revendication 1 comprenant en outre :
une unité de fixation creuse comprenant une extrémité d'injection et une extrémité de connexion en face de l'extrémité d'injection, où l'extrémité de connexion de l'unité de fixation creuse est assemblée de manière détachable à la deuxième extrémité de l'unité extensible ;
une unité de pressage raccordée à l'extrémité d'injection de l'unité de fixation creuse ;
une unité extensible auxiliaire comprenant une extrémité de couplage et une extrémité de manoeuvre en face de l'extrémité de couplage, où l'extrémité de couplage de l'unité extensible auxiliaire est assemblée de manière détachable à l'extrémité d'injection de l'unité de fixation creuse ;
un levier de manoeuvre, qui comprend une extrémité avant et une extrémité arrière, où l'extrémité avant du levier de manoeuvre est assemblée de manière détachable à l'unité extensible ; l'extrémité arrière du levier de manoeuvre est assemblée à l'extrémité de manoeuvre de l'unité extensible auxiliaire, et, par conséquent, elle permet que l'unité extensible auxiliaire puisse ajuster l'unité extensible conformément à la condition d'expansé ou de contracté lorsque le levier de manoeuvre est en fonctionnement ; et
un outillage d'injection, assemblé à l'extrémité d'injection de l'unité de fixation creuse ou à l'extrémité arrière du levier de manoeuvre, de façon à injecter ou pousser un remplisseur médical dans l'unité enveloppante à travers de l'unité de fixation creuse ou à travers du levier de manoeuvre ;
où l'unité extensible auxiliaire est détachée, et l'outillage d'injection est attaché après que l'unité extensible auxiliaire ait étendu l'unité extensible au moyen du levier de manoeuvre pour remplir le remplisseur médical, lorsque le remplissage est fini, l'unité de pressage est activée pour pousser le dispositif de fixation osseuse vers un os dans un site opératoire.
